(19) Europäisches Patentamt
European Patent Office
Office européen des brevets

(11) Veröffentlichungsnummer: **0 270 076 B1**

(12) **EUROPÄISCHE PATENTSCHRIFT**

(45) Veröffentlichungstag der Patentschrift: **09.10.91**

(51) Int. Cl.[5]: **C07D 495/04**, C07D 491/048, //(C07D495/04,333:00,235:00), (C07D491/048,307:00,235:00)

(21) Anmeldenummer: **87117759.8**

(22) Anmeldetag: **01.12.87**

Die Akte enthält technische Angaben, die nach dem Eingang der Anmeldung eingereicht wurden und die nicht in dieser Patentschrift enthalten sind.

# (54) Imidazolderivate und ein Verfahren zu deren Herstellung.

(30) Priorität: **02.12.86 CH 4790/86**

(43) Veröffentlichungstag der Anmeldung: **08.06.88 Patentblatt 88/23**

(45) Bekanntmachung des Hinweises auf die Patenterteilung: **09.10.91 Patentblatt 91/41**

(84) Benannte Vertragsstaaten: **AT BE CH DE ES FR GB IT LI LU NL SE**

(56) Entgegenhaltungen:
**EP-A- 0 092 194**
**EP-A- 0 161 580**
**EP-A- 0 173 185**
**DE-B- 2 058 248**

**PATENT ABSTRACTS OF JAPAN, Band 9, Nr. 324 (C-320)[2047], 19. Dezember 1985**

**CHEMICAL AND PHARMACEUTICAL BULLETIN, Band 32, Nr. 8, 1984, Seiten 3291-3298; K. TANAKA et al.: "Syntheses and antimicrobial activities of five-membered heterocycles having a phenylazo substituent"**

(73) Patentinhaber: **LONZA AG**
**Gampel/Wallis(CH)**

(72) Erfinder: **McGarrity, John, Dr.**
**Rathausgasse 5**
**Visp, Kanton Wallis(CH)**
Erfinder: **Tenud, Leander, Dr.**
**Tirlerstrasse 4**
**Visp, Kanton Wallis(CH)**
Erfinder: **Meul, Thomas, Dr.**
**Balfrinstrasse 21**
**Visp, Kanton Wallis(CH)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Grosse Eschenheimer Strasse 39**
**W-6000 Frankfurt am Main 1(DE)**

**EP 0 270 076 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft neue Imidazolderivate der Formel

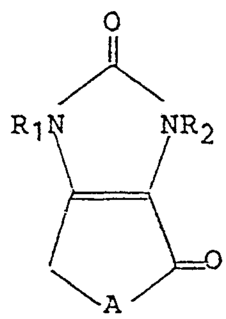

IX

worin $R_1$ eine (R)- oder (S)-1-Phenyl-($C_2$-$C_4$)-alkylgruppe, eine (R)- oder (S)-1-($C_1$-$C_4$)-Alkoxycarbonyl-1-phenylmethylgruppe oder eine (R)- oder (S)-1-Phenoxycarbonyl-1-phenylmethylgruppe oder eine (R)- oder (S)-1-Benzyloxycarbonyl-1-phenylmethylgruppe und $R_2$ Wasserstoff, eine ($C_1$-$C_4$)-Alkylcarbonylgruppe gegebenenfalls substituiert mit Halogen, eine Benzoyl- oder Benzylgruppe jeweils gegebenenfalls substituiert mit Niederalkyl-($C_1$-$C_6$)-gruppen oder Halogen, eine ($C_1$-$C_4$)-Alkoxycarbonylgruppe, eine Phenoxy-carbonylgruppe, eine ($C_1$-$C_4$)-Alkoxy-($C_1$-$C_4$)-alkylgruppe, eine Pyranylgruppe, eine Benzolsulfonylgruppe unsubstituiert oder substituiert mit einer Methylgruppe, eine ($C_1$-$C_4$)-Alkylsulfonylgruppe, eine Diphenyl- oder Dibenzylphosphinylgruppe, eine Di-($C_1$-$C_4$)-alkoxyphosphinylgruppe oder eine Tri-($C_1$-$C_4$)-alkylsilylgruppe bedeutet, und A für ein Schwefel- oder Sauerstoffatom steht.

Diese Verbindungen sind als Zwischenprodukte zur Herstellung von (+)-Biotin geeignet.

Die Herstellung von (+)Biotin kann z.B. erfolgen, indem man eine Verbindung der Formel IX, worin $R_1$ und $R_2$ wie oben definiert sind und A für ein Sauerstoffatom steht, katalytisch mit Wasserstoff hydriert, das erhaltene Diastereomer der Formel

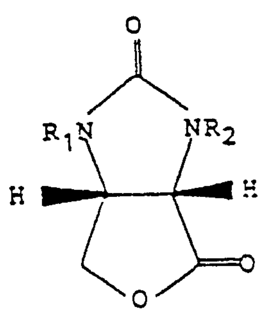

abtrennt, wenn $R^2$ = H durch Umsetzung mit aliphatischen oder aromatischen Säurechloriden, aliphatischen oder aromatischen Carbonsäureanhydriden, Halogenameisensäurealkylestern, Benzylhalogeniden, 1-Alkoxyalkylhalogeniden, Enolether, aromatischen oder aliphatischen Sulfonsäurehalogeniden, Diarylphosphinsäurehalogeniden, Phosphorsäuredialkylesterchloriden, Trialkylsilylhalogeniden oder Trialkylsilylacetamiden eine Schutzgruppe einführt, und weiter auf bekannte Weise durch weitere Umsetzung mit einem Thiocarbonsäuresalzderivat in das entsprechende Thiolacton überführt, dieses entweder mit einer Grignard-Reaktion und anschließender Wasserabspaltung oder mit einer Verbindung der Formel

$(C_6H_5)_3P^{\oplus}(CH_2)_4COOR_3.X^{\ominus}$

worin $R_3$ = H oder Alkyl mit 1 bis 4 C-Atomen und X ein Halogenatom bedeuten, in Gegenwart einer Base zu einer Verbindung der Formel

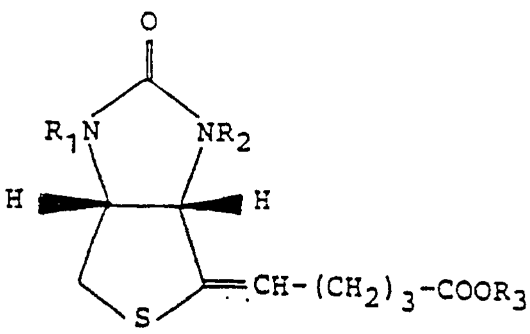

worin $R_3$ oben genannte Bedeutung hat, umsetzt, in einem nächsten Schritt diese Verbindung katalytisch mit Wasserstoff hydriert und schlußendlich durch Abspalten der Schutzgruppen in das Endprodukt überführt.

Aus der US-PS 2 489 232 ist ein Verfahren bekannt, gemäss welchem racemisches Biotin hergestellt wird. Da jedoch bekanntlich nur das optisch aktive (+)-Biotin biologisch aktiv ist, musste das so hergestellte racemische Biotin anschliessend noch in die optischen Antipoden aufgetrennt werden. Einerseits werden hierbei alle Reaktionsstufen mit racemischem Material durchgeführt, wodurch die doppelte Menge an Substanzen verarbeitet werden müssen. Andererseits ist die Spaltung von racemischem Biotin in die entsprechenden Antipoden ein recht kompliziertes Verfahren, welches zudem noch unrentabel ist, da der unerwünschte Antipode praktisch nicht mehr raccmisiert und in den Prozess zurückgeführt werden kann.

Eine Verbesserung dieses Verfahrens ist aus der US-PS 2 489 235 bekannt. Hierbei wird nunmehr die Racematspaltung bereits auf einer früheren Stufe durchgeführt, wobei jedoch auch diesem Verfahren immer noch der Nachteil anhaftet, dass die meisten Reaktionsstufen mit racemischem Material durchgeführt werden und, dass auch hier der bei dieser Spaltung anfallende unerwünschte Antipode ebenfalls praktisch nicht mehr racemisiert und in den Prozess zurückgeführt werden kann.

M.Murakami und Mitarbeiter haben ein verbessertes Verfahren zur Herstellung von dl-Biotin entwickelt (vgl. JP-PS 31 669/1970, 37 775/1970, 37 776/1970 und 3 580/1971). Die Verbesserung besteht in der Einführung einer Carboxybutylgruppe in die 4-Stellung des dl-1,3-Dibenzylhexahydrothieno[3,4-d]-imidazol-2,4-dions. Dieses Dion wird mit einem 1,4-Dihalogenmagnesiumbutan umgesetzt und anschliessend mit Kohlendioxid carboxyliert.

Eine weitere Verbesserung haben Gerecke et al, DE-PS 20 58 248, entwickelt, indem bereits auf einer früheren Stufe durch optische Spaltung eines Triethylaminsalzes der folgenden Formel, worin R einen Cholesterylrest bedeutet, oder eines Ephedrinsalzes der folgenden Formel, worin R einen Cyclohexylrest bedeutet

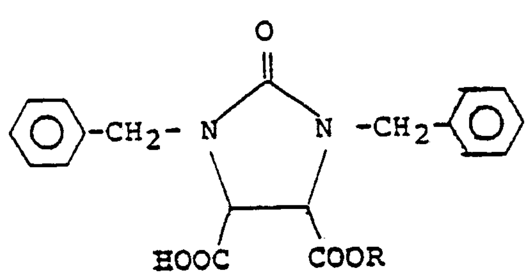

und durch weitere Ueberführung mit Alkalimetallhydriden ein optisch aktives Lacton der Formel

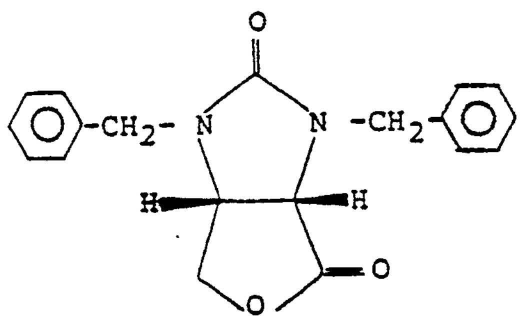

als optisch aktives Zwischenprodukt hergestellt wird. Ein für eine technische Anwendung bedeutender Nachteil besteht in der Anwendung der teuren, optisch aktiven Verbindungen Cholesterin und Ephedrin sowie der teuren Alkalimetallhydriden. Mit dem selben Nachteil, nämlich der Anwendung von teuren opt. aktiven Verbindungen, sind die Verfahren der EP-Anmeldung 0 161 580 und 0 173 185 behaftet.

Es stellte sich somit die Aufgabe, technisch einfach zugängliche Zwischenprodukte zu finden, die einerseits bereits in einer früheren Stufe bezogen auf die Gesamtsynthesen durch einen einfachen Verfahrensschritt optisch gespalten werden können, und andererseits bereits an den entsprechenden Stellen die Konfiguration des Endproduktes aufweisen.

Erfindungsgemäss konnte die Aufgabe gelöst werden mit den neuen Imidazolderivaten der Formel

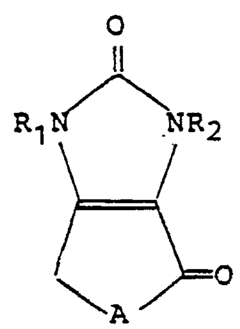

IX

worin $R_1$ und $R_2$ die genannte Bedeutung haben.
Zweckmässige Verbindungen, die unter I fallen, sind die Thienoimidazolderivate der Formel

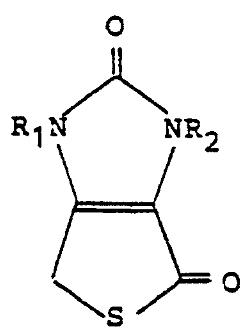

VII

sowie die Furoimidazolderivate der Formel

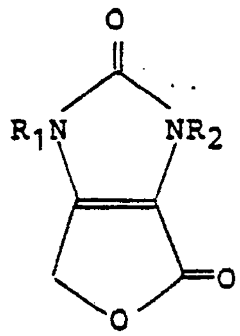

VIII

worin $R_1$ und $R_2$ die genannte Bedeutung haben.

Unter einer 1-Phenyl-($C_2$-$C_4$)-alkylgruppe wird bevorzugt die 1-Phenylethylgruppe verstanden.

Unter der Bezeichnung ($C_1$-$C_4$)-Alkylcarbonyl wird vorzugsweise eine Acetylgruppe verstanden. Als Substituenten der ($C_1$-$C_4$)-Alkylcarbonylgruppe treten vorzugsweise Halogenatome auf. Ein besonders bevorzugter Vertreter der halogenierten ($C_1$-$C_4$)-Alkylcarbonylgruppen ist die Trichloracetylgruppe.

Die Benzoylgruppe sowie die Benzylgruppe sind bevorzugt unsubstituiert. Mit Halogenatomen bzw. Niederalkyl-($C_1$-$C_6$)-gruppen substituierte Vertreter sind aber ebenfalls zweckmäßig. Als solche $R_2$-Substituenten kommen z.B. p-Methoxybenzyl sowie Methylbenzyl in Betracht.

Als bevorzugter Vertreter der substituierten Benzolsulfonylgruppe gilt die p-Toluolsulfonylgruppe.

Unter einer ($C_1$-$C_4$)-Alkylsulfonylgruppe wird vorzugsweise die Methylsulfonylgruppe verstanden.

Generell wird unter den Begriff Alkoxy bzw. Alkyl bevorzugt ($C_1$-$C_4$)-Alkyl(oxy) und unter dem Begriff Aryl bevorzugt unsubstituiertes Benzyl oder Phenyl verstanden.

Erfindungsgemäss werden die Verbindungen, ausgehend von einer Tetronsäure der Formel

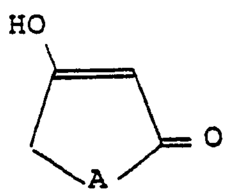

I

worin A die genannte Bedeutung hat, gemäss Anspruch 4 entsprechend dem Schema 1 hergestellt.

Schema 1

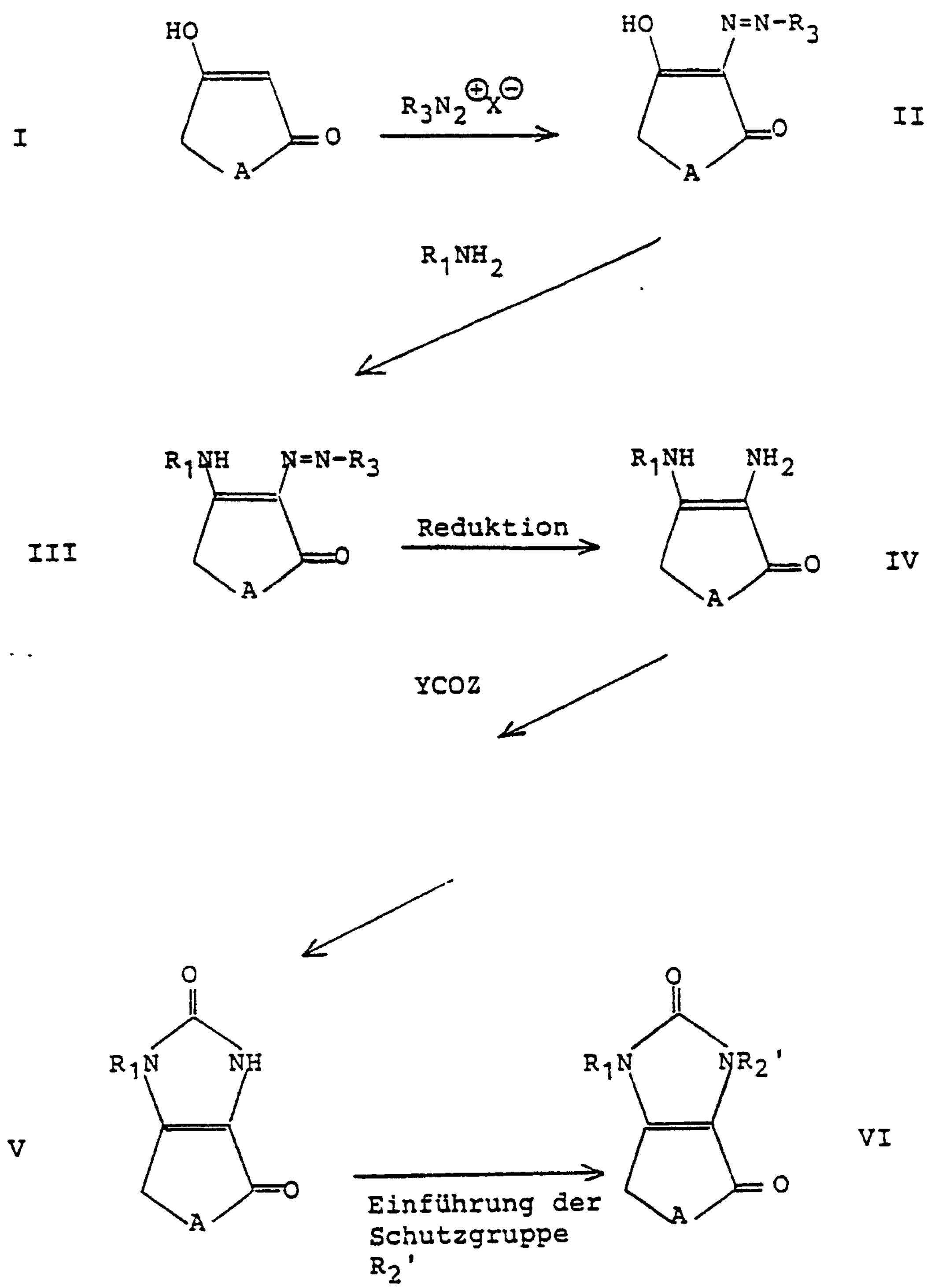


## Beschreibung der einzelnen Verfahrensschritte

I zu II

Die Umsetzung von I zu II beinhaltet eine an und für sich bekannte Diazoherstellung. Für A = O, d.h. für Tetronsäure wurde die Reaktion von Tanaka et al in Chem.Pharm.Bull. 32 (1984) S.3291-3298 beschrieben.
Ueblicherweise wird zuerst ein Diazoniumsalz der Formel

$R_3\ N_2^{\oplus}\ X^{\ominus}$

hergestellt, worin $R_3$ Phenyl, unsubstituiert oder substituiert mit Alkyl-, Halogenalkyl- oder Nitrogruppen oder Halogenatomen, und X Halogen wie Chlor, Brom oder Jod, $BF_4$ oder Hydrogensulfat bedeutet.
Dazu wird auf bekannte Weise ein Anilin der Formel

$R_3$-$NH_2$

vorzugsweise in einer verdünnten wässrigen Mineralsäure wie HCl, $H_2SO_4$ oder $HBF_4$ mit einem Alkalinitrit bei 0 bis 10° C, umgesetzt.
Es ist auch möglich, in Gegenwart von polaren protischen Lösungsmitteln, wie niederen Alkoholen oder Essigsäure, oder mit polaren aprotischen Lösungsmitteln, wie Dimethylsulfoxid, Dimethylformamid, Dimethylacetamid oder Dimethoxyethan, oder im Falle der Anwendung von Diazoniumtetrafluorborat in Acetonitril oder Tetrahydrofuran zu arbeiten.
Nicht ausgeschlossen ist die Anwendung dieser Lösungsmittel als Mischung in unterschiedlichen Verhältnissen mit Wasser.

Das gebildete Diazoniumsalz wird dann mit I, das zweckmässig in Wasser oder in den oben genannten Lösungsmitteln gelöst vorliegt, bei Temperaturen von zweckmässig 0 bis 40° C, vorzugsweise tiefer als 10° C, zu II umgesetzt.

Es ist vorteilhaft, darauf zu achten, dass sich der pH-Wert in einem Bereich von 4 bis 7 befindet. Gegebenenfalls kann der pH-Wert mit einem pH-Korrigens, wie einem Alkalibicarbonat oder einem Phosphatpuffer für wässrige Systeme, oder einem tert.- Amin, auf den gewünschten Bereich eingestellt werden.

Bevorzugt wird die Umsetzung von I zu II mit Benzoldiazoniumchlorid in Wasser durchgeführt.

Die resultierende Phenylazotetronsäuren können in unterschiedlichen Verhältnissen zueinander zusammen mit deren tautomeren Form, dem Phenylhydrazon, vorliegen. Die Aufarbeitung kann auf übliche Weise, vorteilhaft durch Abtrennen des Produktes aus dem Reaktionsgemisch, und gegebenenfalls durch anschliessende Umkristallisation erfolgen.

II zu III

Kennzeichen der Umsetzung von II zu III ist die Einführung einer Aminofunktion in der 4-Position des Heterocyclus mit Hilfe eines chiralen Amins.
Als chirale Amine werden Verbindungen der Formel

$R_1\ NH_2$

worin $R_1$ eine (R)- oder (S)-1-Phenylalkylgruppe oder eine (R)- oder (S)-1-Alkoxycarbonyl-1-phenyl-methylgruppe oder eine (R)- oder (S)-1-Aryloxycarbonyl-1-phenylmethylgruppe bedeutet, eingesetzt. Vorzugsweise wird (R)- oder (S)-1-Phenylethylamin verwendet.

Die Umsetzung wird vorteilhaft in Gegenwart eines sauren Katalysators durchgeführt.

Als Katalysatoren sind vor allem Lewis-Säuren wie die entsprechenden Aluminium-, Bor- oder Titanverbindungen, oder auch Säuren wie Methansulfonsäure oder p-Toluolsulfonsäure geeignet. Geeignete Lewis-Säuren sind Bortrifluoridethyletherat, Trimethylborat, Triethylborat, Titantetrachlorid, Titantetraisopropoxid oder Aluminiumchlorid.

Zweckmässig wird der Katalysator in Mengen von 1 bis 50 Mol%, vorzugsweise in Mengen von 1 bis 20 Mol%, eingesetzt.

Da mit der Reaktion eine Wasserabspaltung einhergeht, werden als Lösungsmittel zweckmässig entweder Wasserschleppmittel wie Toluol oder Benzol, oder Tetrahydrofuran, Acetonitril, Dioxan, Dimethylformamid, Dimethylacetamid, chlorierte Kohlenwasserstoffe wie Chloroform oder Methylenchlorid, auch niedere Alkohole wie Methanol, Ethanol oder Propanol, zweckmässig zusammen mit den üblichen Trocknungsmitteln wie Molekularsieben, Natriumsulfat, Calciumsulfat oder Magnesiumsulfat, eingesetzt.

Zweckmässig wird bei Temperaturen zwischen 20 bis 120° C gearbeitet. Es ist auch von Vorteil, die

Reaktion in einer Inertgasatmosphäre (z.B. Stickstoff oder Argon) durchzuführen.

III kann dann nach fachmännischen Methoden, z.B. durch Eindampfen und anschliessende Umkristallisation, aufgearbeitet und isoliert werden.

III zu IV

Die Reduktion von III zu IV kann entweder mittels einer katalytischen Hydrierung mit Wasserstoff oder aber durch Umsetzung mit Zink in Gegenwart von Essigsäure oder mit Salzsäure erfolgen. Vorzugsweise wird die Reduktion mittels einer katalytischen Hydrierung mit Wasserstoff erreicht. Als Hydrierkatalysatoren werden zweckmässig Platin, Palladium, Rhodium, Ruthenium oder Raney-Nickel, gegebenenfalls auf Trägermaterialien wie Kohle, Tonerde, Bimsstein, Aluminiumoxid, Aluminiumsilikat verwendet. Vorzugsweise wird Platin auf Kohle als Trägermaterial eingesetzt.

Die Katalysatormenge wird zweckmässig zwischen 5 bis 20 Mol% gewählt. Die Menge Katalysator auf dem Trägermaterial kann zwischen 1 bis 10% variieren.

Es ist vorteilhaft, in Gegenwart eines Lösungsmittels zu arbeiten. Geeignet sind Essigsäurealkylester wie der Essigsäureethylester, Ether wie Tetrahydrofuran, Dioxan oder Dimethoxyethan, oder auch Dimethylformamid, Dimethylacetamid, Acetonitril, Essigsäure oder auch niedere Alkohole. Besonders gut einsetzbar sind Essigsäureethylester, Tetrahydrofuran oder Ethanol.

Man arbeitet zweckmässig bei einem Wasserstoffdruck zwischen 1 und 50 bar, vorzugsweise zwischen 20 und 40 bar, und bei Temperaturen zwischen zweckmässig 0 und 60 °C, vorzugsweise zwischen 10 und 30 °C.

Nach Abfiltrieren des Katalysators kann das Reaktionsgemisch auf übliche Weise, z.B. durch Ausfällen des Produktes in einem sehr unpolaren Lösungsmittel, aufgearbeitet werden.

IV zu V

Zur Imidazolbildung wird IV mit Phosgen oder einem Phosgenäquivalent behandelt.
Als Phosgenäquivalent werden zweckmässig Verbindungen der Formel

YCOZ

worin Y und Z Imidazolyl oder Chlor bedeuten, oder Y Chlor und Z Alkoxy, substituiert oder unsubstituiert, oder Aryloxy, substituiert oder unsubstituiert, bedeuten, eingesetzt.
Vorteilhafte Aequivalente sind die Chlorameisensäureniederalkylester, der Chlorameisensäurephenylester, der Chlorameisensäurebenzylester oder das Carbonyldiimidazolid.

Die Umsetzung verläuft zweckmässig in Gegenwart einer Base. Zur Anwendung können zweckmässig tertiäre aliphatische Amine, wie Triethylamin, aromatische und cyclische tertiäre Amine, wie Pyridin oder Diazabicyclooctan, oder aber auch anorganische Basen gelangen. Vorzugsweise werden aber tertiäre aliphatische Amine, wie Triethylamin, eingesetzt.
Der Einsatz einer Base erübrigt sich, wenn IV mit Carbonyldiimidazolid als Base umgesetzt wird.

Es ist von Vorteil, die Reaktion in Gegenwart von Ethern wie Tetrahydrofuran oder Dioxan, halogenierten Kohlenwasserstoffen wie Chloroform oder Methylenchlorid, aromatischen Kohlenwasserstoffen wie Toluol, Carbonsäureamiden wie Dimethylformamid, Essigsäurealkylestern, oder auch in Acetonitril als inerte organische Lösungsmittel durchzuführen.

Die Reaktionstemperatur wählt man zweckmässig in einem Bereich von 0 bis 80 °C.

Die Aufarbeitung erfolgt auf übliche Weise durch Abtrennen der entstandenen Salze, Abdampfen des Lösungsmittels und gegebenenfalls durch Reinigung des Produktes durch beispielsweise eine Umkristallisation.

V zu VI

An und für sich kann bereits V für die stereoselektive Weiterumsetzung eingesetzt werden. Gegebenenfalls kann aber das Wasserstoffatom in 3-Position des Imidazolrings durch eine Schutzgruppe $R_2'$ ersetzt werden.
Die Einführung der Schutzgruppe $R_2'$ erfolgt zweckmässig durch Umsetzung von V mit substituierten oder unsubstituierten, aliphatischen oder aromatischen Säurehalogeniden, wie z.B. Acetylchlorid, Propionylchlorid, Benzoylchlorid, p-Toluolsulfonsäurechlorid oder Benzylchlorid, mit Chlorameisensäureestern, wie Chlorameisensäureethylester, Chlorameisensäure-tert.butylester, Chlorameisensäurebenzylester oder Chlorameil-

sensäurephenylester, mit Phosphorverbindungen, wie Diphenylphosphinsäurechlorid oder Phosphorsäurediethylesterchlorid, mit aromatischen oder aliphatischen Sulfonsäurehalogeniden wie Methansulfonsäurechlorid, p-Toluolsulfonsäurechlorid oder Benzolsufonsäurechlorid, mit Silylverbindungen, wie Bis-(trimethylsilyl)-acetamid, oder tert.Butyl-dimethylsilylchlorid, mit Alkoxyalkylhalogeniden wie Methoxymethylchlorid, oder mit Enolethern wie Dihydropyran. Ebenso geeignet sind die substituierten oder unsubstituierten, aliphatischen oder aromatischen Carbonsäureanhydride, wie Essigsäureanhydrid.

Das Einführen der Schutzgruppen kann nach bekannten Methoden erfolgen. Infolgedessen wird nicht näher darauf eingegangen.

Nach diesem erfindungsgemässen 4-stufigen, bei Einführung einer Schutzgruppe 5-stufigen Verfahren kann auf einfache Weise und in guter Ausbeute zu der Zwischenstufe IX gelangt werden. Diese besitzt einerseits bereits das Gerüst des Endproduktes und gestattet auf einfache Weise den Einbau der asymmetrischen Zentren von Biotin.

Beispiel 1

a) Herstellung von 3-Phenylazotetronsäure
(3-Phenylazo-4-hydroxyfuran-2(5H)-on)

Man gab in einem 1,5 l Sulfierkolben, der mit einem 250 ml Tropftrichter, einem mechanischen Rührer und einem Thermometer ausgerüstet wurde, 300 ml Salzsäurelösung 6N. Mit Eiskühlung tropfte man 57,6 g destilliertes Anilin (0,61 Mol) zu. Zur gebildeten Suspension gab man tropfenweise eine Lösung von 43,92 g Natriumnitrit (0,64 Mol) in 90 ml Eiswasser und rührte während 40 Min.
Die so gebildete Diazoniumsalz-Lösung wurde in eine Lösung von 60 g Tetronsäure (0,6 Mol) und 120 g Natriumacetat-trihydrat (0,88 Mol) in 900 ml Wasser während 30 Min. zugetropft.
Nach dieser Zugabe fiel sofert ein gelber Feststoff aus. Man rührte die Reaktionsmischung bei 10 °C während 1,5 Std., filtrierte ab und wusch das Produkt mit 500 ml kaltem Methanol. Bei 35 °C wurde im Vakuum getrocknet.
Ausbeute: 113,2 g = 92,4%,
Smp.: 199-200 °C (zersetzt).

b) Herstellung von 3-Phenylazo-4-[(S)-(1-phenylethylamino)]-furan-2(5H)-on

In einem 500 ml Dreihalskolben, der mit einem Wasserabscheider, einem Thermometer und einem Magnetrührer ausgerüstet war, suspendierte man 20,0 g 3-Phenylazotetronsäure (98 mMol) in 190 ml Toluol und erhitzte unter Argon auf 80 °C. Dann gab man 13,1 g (S)-Phenylethylamin (108 mMol) und 2,8 g Triethylborat (19 mMol) zu. Unter Vakuum von 300 mbar brachte man das Lösungsmittel zum Rückfluss. Nach 7 Std. wurde das Toluol abgedampft. Der schwarze Rückstand wurde mit 100 ml Ether gewaschen, bis eine braune Masse ausfiel. Diese wurde in Ether zerkleinert und man erhielt ein gelbliches Produkt.
Das Produkt 3-Phenylazo-4-[(S)-(1-Phenylethylamino)]-furan-2(5H)-on wurde abfiltriert und im Vakuum getrocknet.
Ausbeute: 28,36 g = 94,0%,
Smp.: 114-115 °C.
NMR: ($CDCl_3$, 300 MHz) $\delta$ in ppm
1,69, d, J = 7 Hz, 3H
4,42, d, J = 16 Hz, 1H
4,54, bm, 1H
4,81, d, J = 16 Hz, 1H
7,26-7,45, m, 8H
7,78, d, J = 8 Hz, 2H
10,55, bs, 1 H
MS: (E.I. 70 ev) m/e 307 (9%) $M^+$, 195 (25%), 171 (11%), 126 (10%), 105 (100%), 93 (28%)
IR (KBr) $cm^{-1}$ 3064, 3026, 1746 (s), 1621 (s), 1456, 1356, 1288, 1045, 756,
UV (MeOH) $\lambda_{max.}$
366 nm ($\epsilon$ = 21,050)
260 nm ($\epsilon$ = 11,540)
235 nm ($\epsilon$ = 12,800)
Elementaranalyse für $C_{18}H_{17}N_3O_2$ (307,35)
ber. C 70,3% H 5,6% N 13,7%

gef. C 70,3% H 5,5% N 13,4%
$[\alpha]_D^{25}$ [c=1 $CHCl_3$] +785°

c) Herstellung von 3-Amino-4-[(S)-(1-phenylethylamino)]-furan-2(5H)-on

In einem 500 ml Autoklav legte man 13,50 g 3-Phenylazo-4-[(S)-(1-phenylethylamino)]-furan-2(5H)-on (44 mMol), 133 ml Essigsäureethylester und 0,77 g Platin auf Kohle (5%) vor. Der Autoklav wurde geschlossen und zweimal unter Rühren mit Wasserstoff gespült. Nun wurde die Reaktionsmischung mit Wasserstoff unter 40 bar Druck während 30 Min. hydriert. Der Katalysator wurde unter Argon abfiltriert und die Mutterlauge tropfenweise und unter Eiskühlung mit 130 ml Oktan versetzt.
3-Amino-4-[(S)-(1-phenylethylamino)]-furan-2(5H)-on fiel in Form von beigen Kristallen aus. Es wurde unter Vakuum bei Raumtemperatur getrocknet.
Ausbeute: 8,53 g = 89,0%,
Smp.: 127,5-128,0° C.
NMR: ($CDCl_3$, 300 MHz) δ in ppm
1,55, d, J = 7,0 Hz, 3H
2,35, bs, 2H
4,21, d, J = 15 Hz, 1H
4,51, d, q, J = 7 Hz, 7 Hz, 1H
4,53, d, J = 15 Hz, 1H
4,83, bd, J = 7 Hz, 1H
7,25-7,4, m, 5H
MS (E.I. 70 ev) m/e 218 (10%) $M^+$, 114 (18%), 105 (100%).
IR (KBr) $cm^{-1}$ 3424, 3341 (s), 1737, 1651, 1584, 1428, 700.
UV (MeOH) $\lambda_{max.}$ 283 nm (ε = 16,610)
Elementaranalyse für $C_{12}H_{14}N_2O_2$ (218,26)
ber. C 66,0% H 6,5% N 12,8%
gef. C 66,2% H 6,4% N 12,8%
$[\alpha]_D^{25}$ [c=1 $CHCl_3$] +20,5°

d) Herstellung von 1-[(S)-1-Phenylethyl)]-1H-furo-[3,4-d]-imidazol-2,4(3H,6H)-dion

In einem 50 ml Dreihalskolben, der mit einem 50 ml Tropftrichter und Magnetrührer ausgerüstet war, legte man 8,06 g 3-Amino- 4-[(S)-(1-phenylethylamino)]-furan-2(5H)-on (36 mMol) und 65 ml Tetrahydrofuran vor und kühlte auf 0° C. Dann tropfte man während 40 Min. eine Lösung von 5,78 g Chlorameisensäurephenylester (36 mMol) in 10 ml Tetrahydrofuran und gleichzeitig eine Lösung von 3,78 g Triethylamin (36 mMol) in 10 ml Tetrahydrofuran zu. Die weisse Suspension wurde abfiltriert und die leicht braune Mutterlauge abgedampft. Der Rückstand, ein brauner Schaum, wurde in 60 ml Acetonitril aufgelöst und diese Lösung während 40 Minuten zu einer Lösung von 3,78 g Triethylamin (36 mMol) in 40 ml Acetonitril, die zum Rückfluss erhitzt wurde, zugetropft. Das Reaktionsgemisch wurde abgedampft und der Rückstand mit 50 ml Ether gewaschen.
Das beige Produkt [1-(S)-(1-Phenylethyl)]-1H-furo-[3,4-d]-imidazol-2,4(3H,6H)- dion wurde abfiltriert und im Vakuum getrocknet.
Nach Umkristallisation in Methanol betrug die Ausbeute 5,75 g = 66,0%.
Smp.: 159,5-160° C.
NMR: ($CDCl_3$, 300 MHz) δ in ppm
1,77, d, J = 7 Hz, 3H
4,07, d, J = 16 Hz, 1H
4,72, d, J = 16 Hz, 1H
5,57, q, J = 7 Hz, 1H
7,35-7,58, m, 5H
9,75, bs, 1H
MS (E.I. 70 ev) m/e 244 (16%), 105 (100%), 77 (37%).
IR (KBr) $cm^{-1}$ 3250, 2981, 1761, 1700, 1482, 1450, 1340, 1268, 1000, 739, 705
UV (MeOH) $\lambda_{max.}$ 266 nm (ε = 12,900)
Elementaranalyse für $C_{13}H_{12}O_3N_2$ (244,25)
ber. C 63,9% H 4,9% N 11,5%
gef. C 63,6% H 4,9% N 11,3%

$[\alpha]_D^{25}$ [c=1 $CHCl_3$] -69,5°

Beispiel 2

a) Herstellung von 3-Phenylazothiotetronsäure
(3-Phenylazo-4-hydroxythiophen-2(5H)-on oder 2,3,4-Trioxotetrahydrothiophen-3-phenylhydrazon)

Man legte in ein 100 ml Becherglas, das mit einem 100 ml Tropftrichter, einem Thermometer und einem mechanischen Rührer ausgerüstet war, 28 ml Salzsäurelösung 6N vor. Mit Eiskühlung tropfte man 5,02 g Anilin (53,9 mMol) zu. Dann gab man unter starkem Rühren zur gebildeten Suspension tropfenweise eine Lösung von 3,81 g Natriumnitrit (55,2 mMol) in 21 ml Eiswasser während 30 Min. zu. Die so gebildete Diazoniumsalz-Lösung wurde zu einer Lösung von 5,78 g Thiotetronsäure (50 mMol) in 49 ml 1N Natronlauge bei 5°C unter starkem Rühren während 30 Min. zugetropft. Gleichzeitig wurden 55 ml 1N Natriumbicarbonat-Lösung zugetropft, um den pH-Wert von 7,0 konstant zu halten.
Das senfgelbe Produkt wurde abfiltriert, mit 30 ml Wasser gewaschen und im Vakuum getrocknet.
Nach Umkristallisation in Toluol betrug die Ausbeute 10,5 g = 95,0%.
Smp.: 195-196,5°C.
NMR: ($CDCl_3$, 300 MHz) δ in ppm
3,89, s, 2H
3,95, s, 1H
7,32, t, J = 7 Hz, 2H
7,46, t, J = 7 Hz, 2H
7,58, d, J = 7 Hz, 2H
3,89, s, 2H
6,67, s, 1H
7,32, t, J = 7 Hz, 1H
7,45, t, J = 7 Hz, 2H
7,57, d, J = 7 Hz, 2H
Das Tautomerverhältnis von 3-Phenylazothiotetronsäure zu 2,3,4-Trioxotetrahydrothiophen-3-phenylhydrazon ist 3 zu 1.
MS (E.I. 70 ev) m/e 220 (70%) $M^+$, 143 (13%), 105 (31%), 92 (30%), 77 (100%)
IR (KBr) $cm^{-1}$ 3450, 1688, 1673 s, 1532 s, 1465, 1424, 1397 s, 1129 s, 912 s, 764 s,
UV (MeOH) $\lambda_{max.}$
408 nm (ε = 14,100)
372 nm (ε = 16,700)
235 nm (ε = 6,670)
Elementaranalyse für $C_{10}H_8N_2O_2S$ (220,25)
ber. C 54,5% H 3,7% N 12,7% S 14,6%
gef. C 54,3% H 3,5% N 12,7% S 14,8%

b) Herstellung von 3-Phenylazo-4-[(S)-(1-phenylethylamino)]-thien-2(5H)-on

In einem 250 ml Dreihalskolben, der mit einem Wasserabscheider, einem Intensivkühler und einem Magnetrührer ausgerüstet war, löste man unter Stickstoff 6,56 g 3-Phenylazothiotetronsäure (29,8 mMol) in 165 ml Toluol unter Rückfluss. Dann gab man 14,53 g S-1-Phenylethylamin (119,9 mMol) zu und dann während 40 Min. ein Lösung von 2,19 g Bortrifluoridethyletherat in 5 ml Toluol. Nun liess man das Reaktionsgemisch auf Raumtemperatur abkühlen. Dann extrahierte man dieses Reaktionsgemisch mit 100 ml 0,9N Salzsäure, danach mit 50 ml gesättigter Natriumbicarbonat-Lösung und abschliessend mit 50 ml gesättigter Natriumsulfat-Lösung.
Die dunkelbraune Lösung wurde über 20 g Magnesiumsulfat getrocknet und abgedampft. Zu dem braunen, dickflüssigen Rückstand gab man 50 ml Ether und liess unter schwachem Vakuum rotieren. Der so gebildete Feststoff wurde in 6 ml Dichlormethan unter Rückfluss gelöst und nach einer Zugabe von 14 ml Ether bei 0°C umkristallisiert.
Nach einer weiteren Umkristallisation betrug die Ausbeute an 3-Phenylazo-4-[(S)-(1-phenylethylamino)]-thien-2(5H)-on 5,59 g = 58%. Smp. 129-130°C.
NMR: ($CDCl_3$, 300 MHz) δin ppm
1,71, d, J = 7 Hz, 3H
3,64, d, J = 17 Hz, 1H

3,98, d, J = 17 Hz, 1H
4,77, d, q, J = 7 Hz, 7 Hz, 1H
7,25-7,5, m, 8H
7,76, d, J = 8 Hz, 2H
12,34, bs, 1H
MS (E.I. 70 ev) m/e 323 (10%) $M^+$, 195 (22%), 105 (100%), 93 (30%), 77 (25%),
IR (KBr) $cm^{-1}$ 3500 b, 1720, 1600 s, 1580 s, 1450, 1280,
UV (MeOH) $\lambda_{max.}$
410 nm ($\epsilon$ = 9,600)
375 nm ($\epsilon$ = 21,910)
290 nm ($\epsilon$ = 11,880)
231 nm ($\epsilon$ = 13,823)
Elementaranalyse für $C_{18}H_{17}N_3OS$ (323,41)
ber. C 66,8% H 5,3% N 13,0% S 9,9%
gef. C 66,7% H 5,2% N 13,2% S 9,5%
$[\alpha]_D^{25}$ [c=1 $CHCl_3$] +889°

c) Herstellung von 3-Amino-4-[(S)-(1-phenylethylamino)]-thien-2(5H)-on

In einem 100 ml Autoklav legte man eine Lösung von 5,0 g 3-Phenylazo-4-[(S)-(1-phenylethylamino)]-thien-2(5H)-on (15,5 mMol) in 30 ml Tetrahydrofuran vor. Dazu gab man 0,49 g Platin auf Kohle 5%. Man spülte den Autoklav zweimal und hydrierte die Lösung mit einem Wasserstoffdruck von 30 bar während 45 Min. Der Katalysator wurde unter Argon abfiltriert und die Mutterlauge mit 90 ml Hexan tropfenweise unter Eiskühlung versetzt. 3-Amino-4-[(S)-(1-phenylethylamino)]-thien-2(5H)-on fiel als beiges, dichflüssiges Oel aus.
Ausbeute: 2,4 g = 65,0%.
NMR: ($CDCl_3$, 300 MHz) $\delta$ in ppm
1,54, d, J = 7 Hz, 3H
3,30, bs, 3-4H
3,37, d, J = 16,5 Hz, 1H
3,72, d, J = 16,5 Hz, 1H
4,60, q, J = 7 Hz, 1H
7,22-7,37, m, 5H
MS (E.I. 70 ev) m/e 234 (4%) $M^+$, 130 (18%), 105 (100%)

d) Herstellung von (S)-(1-Phenylethyl)-1H-thieno]-3,4-d]-imidazol-2,4(3H,6H)-dion

In einen 250 ml Dreihalskolben, der mit zwei 50 ml Tropftrichtern, mit einem Thermometer und einem Magnetrührer ausgerüstet war, legte man 22 ml Tetrahydrofuran vor. Man kühlte bis 0°C und gab unter Argon 11,1 ml 1,25M Phosgenlösung in Toluol (13,87 mMol) zu. Gleichzeitig tropfte man eine Lösung von 3,24 g 3-Amino-4-[(S)-(1-phenylethylamino)]-thien-2(5H)-on (13,82 mMol) in 10 ml Tetrahydrofuran und eine Lösung von 2,18 g Triethylamin (27,75 mMol) in 10 ml Tetrahydrofuran während 3 Std. bei 5°C zu. Dazu gab man 10 ml 5% wässrige Ammoniak-Lösung. Das Tetrahydrofuran wurde abgedampft und der wässrige Rückstand wurde dreimal mit 10 ml Dichlormethan extrahiert. Die Lösung wurde eingeengt und durch 100 g Kieselgel mit 700 ml Essigsäureethylester chromatographiert.
Die Ausbeute an (S)-(1-Phenylethyl)-thieno-[3,4-d]-imidazol-2,4(3H,6H)-dion (beige Kristalle) betrug 2,16 g = 60%.
Smp.: 218-220°C.
NMR: ($CDCl_3$, 300 MHz) $\delta$ in ppm
1,83, d, J = 7 Hz, 3H
3,23, d, J = 16,5 Hz, 1H
3,86, d, J = 16,5 Hz, 1H
5,73, q, J = 7 Hz, 1H
7,40, m, 5H
8,78, bs, 1H
MS (E.I. 70 ev) m/e 260 (4%) $M^+$, 156 (4%), 105 (100%), 79 (10%), 77 (12%).
IR (KBr) $cm^{-1}$ 3225, 2945, 2918, 1702 s, 1619, 1451, 1351, 1268
UV (MeOH) $\lambda_{max.}$

297 nm, ($\epsilon$ = 9,805)
248 nm ($\epsilon$ = 5,960)
Elementaranalyse für $C_{13}H_{12}N_2O_2S$ (260,31)
ber. C 60,0% H 4,7% N 10,7% S 12,3%
gef. C 59,6% H 4,7% N 10,8% S 12,0%
$[\alpha]_D^{25}$ [c=1 $CHCl_3$] -63,2°

e) Herstellung von 1-[(S)-(1-Phenylethyl)]-3-acetyl-1H-thieno-[3,4-d]-imidazol-2,4 (3H,6H)-dion

In einem 25 ml Kolben erhitzte man 0,5 g 1-[(S)-(1-Phenylethyl)]-1H-thieno-[3,4-d]-imidazol-2,4(3H,6H)-dion (1,94 mMol) in 20 ml Essigsäureanhydrid bei 50°C während 3 Std. Dann dampfte man das Lösungsmittel ab und wusch den Rückstand mit 3 ml Ether. Das beige Produkt wurde darauf getrocknet.
Die Ausbeute an 1-[(S)-(1-Phenylethyl)]-3-acetyl-1H-thieno-[3,4-d]-imidazol-2,4(3H,6H)-dion betrug 0,43 g = 73,0%.
Smp. 187-189,5°C.
NMR: ($CDCl_3$, 300 MHz) $\delta$ in ppm
1,85, d, J = 7 Hz, 3H
2,71, s, 3H
3,18, d, J = 17,5 Hz, 1H
3,83, d, J = 17,5 Hz, 1H
5,71, q, J = 7 Hz, 1 H
7,35-7,45, m, 5H
MS (E.I. 70 ev) m/e 302 (1%) $M^+$, 260 (10%) ($-CH_2CO$), 165 (5%), 105 (100%), 43 (20%).
IR (KBr) $cm^{-1}$ 2920, 1736 s, 1447, 1376, 1354, 1298.
UV (MeOH) $\lambda_{max.}$
297 nm ($\epsilon$ = 11,480)
248 nm ($\epsilon$ = 6,930)
Elementaranalyse für $C_{15}H_{14}O_3N_2S$ (302,35)
ber. C 59,6% H 4,7% N 9,3% S 10,6%
gef. C 58,9% H 4,7% N 9,2% S 10,3%
$[\alpha]_D^{25}$ [c=1 $CHCl_3$] -63,3°

Beispiel 3

Herstellung von 1-[(S)-(1-Phenylethyl)]-3-benzyl-1H-thieno-[3,4-d]-imidazol-2,4(3H,6H)-dion

Zu einer Suspension von 75 mg Natriumhydrid (3,1 mMol) in 15 ml Tetrahydrofuran gab man 0,73 g 1-[(S)-(1-Phenylethyl)]-1H-thieno-[3,4-d]-imidazol-2,4(3H,6H)-dion (2,8 mMol), 0,54 g Benzylbromid (3,2 mMol) und 10 ml Diethylenglycoldiethylether. Das Reaktionsgemisch wurde unter Rückfluss während 12 Std. erhitzt. Die Lösungsmittel wurden im Vakuum abgedampft und der Rückstand zwischen 10 ml Dichlormethan und 10 ml Wasser getrennt. Die wässrige Phase wurde zweimal mit 10 ml Dichlormethan gewaschen. Die organischen Phasen wurden vereinigt, mit 10 g Magnesiumsulfat getrocknet und abgedampft. Der feste Rückstand wurde mit 5 ml Ether gewaschen, abfiltriert und getrocknet.
Die Ausbeute an 1-[(S)-(1-Phenylethyl)]-3-benzyl-1H-thienol-[3,4-d]-imidazol-2,4(3H,6H)-dion betrug 57,0 mg = 60%.
Smp.: 143-145°C.
NMR: ($CDCl_3$, 300 MHz) $\delta$ in ppm
1,79, d, J = 7 HZ, 3H
3,18, d, J = 17 Hz, 1H
3,78, d, J = 17 Hz, 1H
5,03, s, 2H
5,21, J = 7 Hz, 1H
7,27-7,4, m, 8H
7,49, d, J = 8 Hz, J = 1,5 Hz, 2H
MS (E.I. 70 ev) m/e 350 (4%) $M^+$, 246 (12%), 105 (100%), 91 (40%).
IR (KBr) $cm^{-1}$ 2982, 1707 s, 1672 s, 1456, 1346, 846, 700.
UV (MeOH) $\lambda_{max.}$ 285,8 nm ($\epsilon$ = 10,200)

## Patentansprüche

1. Imidazolderivate der Formel

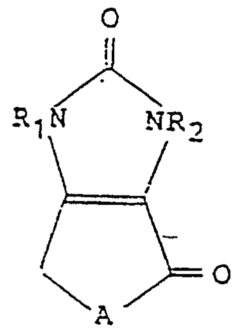

IX

worin $R_1$ eine (R)- oder (S)-1-Phenyl-($C_2$-$C_4$)-alkylgruppe, eine (R)- oder (S)-1-($C_1$-$C_4$)-Alkoxycarbonyl-1-phenylmethylgruppe oder eine (R)- oder (S)-1-Phenoxycarbonyl-1-phenylmethylgruppe oder eine (R)- oder (S)-1-Benzyloxycarbonyl-1-phenylmethylgruppe und $R_2$ Wasserstoff, eine ($C_1$-$C_4$)-Alkylcarbonylgruppe gegebenenfalls substituiert mit Halogen, eine Benzoyl- oder Benzylgruppe jeweils gegebenenfalls substituiert mit Niederalkyl-($C_1$-$C_6$)-gruppen oder Halogen, eine ($C_1$-$C_4$)-Alkoxycarbonylgruppe, eine Phenoxy-carbonylgruppe, eine ($C_1$-$C_4$)-Alkoxy-($C_1$-$C_4$)-alkylgruppe, eine Pyranylgruppe, eine Benzolsulfonylgruppe unsubstituiert oder substituiert mit einer Methylgruppe, eine ($C_1$-$C_4$)-Alkylsulfonylgruppe, eine Diphenyl- oder Dibenzylphosphinylgruppe, eine Di-($C_1$-$C_4$)-alkoxyphosphinylgruppe oder eine Tri-($C_1$-$C_4$)-alkylsilylgruppe bedeutet, und A für ein Schwefel- oder Sauerstoffatom steht.

2. Verfahren zur Herstellung von Imidazolderivaten nach Patentanspruch 1, dadurch gekennzeichnet, dass man eine Tetronsäure der Formel

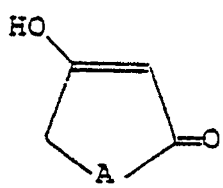

I

worin A die genannte Bedeutung hat, mit einem Diazoniumsalz umsetz, die entstandene Arylazotetronsäure resp. das tautomere Arylhydrazon in einem weiteren Schritt mit einem chiralen Amin in eine Arylazoaminoverbindung überführt, diese reduziert, das entstandene Diamin mit Phosgen oder einem phosgenäquivalenten Reagens in das entsprechende Imidazol überführt und gegebenenfalls durch Umsetzung mit substituierten oder unsubstituierten, aliphatischen oder aromatischen Säurechloriden, aliphatischen oder aromatischen Carbonsäureanhydriden, Halogenaemeisensäurealkylestern, 1-Alkoxyalkylhalogeniden, Enolethern, aromatischen oder aliphatischen Sulfonsäurehalogeniden, Diarylphosphinsäurehalogeniden, Phosphorsäuredialkylesterhalogeniden, Trialkylsilylhalogeniden oder Trialkylsilylacetamiden eine Schutzgruppe einführt.

3. Verfahren nach Anspruch 2, dadurch gekennzeichnet, dass man als Diazoniumsalze substituierte Benzoldiazoniumhalogenide, -bortetrafluoride oder -hydrogensulfate einsetzt.

4. Verfahren nach einem oder beiden der Ansprüche 2-3, dadurch gekennzeichnet, dass man als chirale Amine die (R)- oder (S)-1-Phenylalkylamine oder die (R)- oder (S)-2-Aminophenylessigsäurealkylester einsetzt.

5. Verfahren nach einem oder mehreren der Ansprüche 2-4, dadurch gekennzeichnet, dass man für die Ueberführung in die Arylazoaminoverbindungen saure Katalysatoren verwendet.

6. Verfahren nach einem oder mehreren der Ansprüche 2-5, dadurch gekennzeichnet, dass die Reduktion durch katalytische Hydrierung mit Wasserstoff oder durch Umsetzung mit Zink in Gegenwart von Salz- oder Essigsäure erfolgt.

7. Verfahren nach Anspruch 6, dadurch gekennzeichnet, dass die Reduktion durch Hydrierung in Gegenwart von Platin, Palladium, Rhodium, Ruthenium oder Raney-Nickel, gegebenenfalls auf Trägermaterialien wie Kohle, Tonerde, Bimsstein, Aluminiumoxid oder Aluminiumsilikat, erfolgt.

8. Verfahren nach einem oder mehreren der Ansprüche 2-7, dadurch gekennzeichnet, dass man als Phosgenäquivalente Verbindung der Formel

YCOZ

einsetzt, worin Y und Z gleich sind und Chlor oder Imidazolyl bedeuten, oder Y Chlor und Z Alkoxy substituiert oder unsubstituiert oder Aryloxy substituiert oder unsubstituiert, bedeuten.

9. Verfahren nach einem oder mehreren der Ansprüche 2-8, dadurch gekennzeichnet, dass man die Umsetzung zum Imidazol in Gegenwart eine Base durchführt.

10. Verfahren nach Anspruch 9, dadurch gekennzeichnet, dass man als Base ein tert. Amin verwendet.

## Claims

1. Imidazole derivatives of the formula

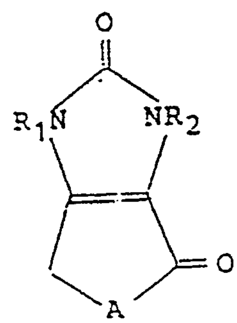

IX

wherein $R_1$ is an (R)- or (S)-1-phenyl-($C_2$-$C_4$)-alkyl group, an (R)- or (S)-1-($C_1$-$C_4$)-alkoxycarbonyl-1-phenyl methyl group or an (R)- or (S)-1-phenoxycarbonyl-1-phenyl methyl grcup or an (R)-or (S)-1-benzyloxycarbonyl-1-phenyl methyl group, and $R_2$ is hydrogen, a ($C_1$-$C_4$)-alkylcarbonyl group, if desired substituted with halogen, a benzoyl or benzyl group, each if desired substituted with lower alkyl-($C_1$-$C_6$)-groups or halogen, a ($C_1$-$C_4$)-alkoxycarbonyl group, a phenoxycarbonyl group, a ($C_1$-$C_4$)-alkoxy-($C_1$-$C_4$)-alkyl group, a pyranyl group, a benzene sulfonyl group, unsubstituted or substituted with a methyl group, a ($C_1$-$C_4$)-alkyl sulfonyl group, a diphenyl or dibenzyl phosphinyl group, a di-($C_1$-$C_4$)-alkoxyphosphinyl group or a tri-($C_1$-$C_4$)-alkyl silyl group, and A is a sulfur or oxygen atom.

2. Process for the preparation of imidazole derivatives according to patent claim 1, characterized in that a tetronic acid of the formula

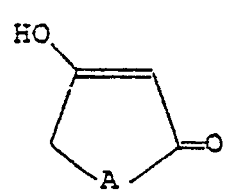

I

wherein A has the mentioned meaning, is reacted with a diazonium salt, the resulting aryl azotetronic acid or the tautomeric aryl hydrazone is converted with a chiral amine in a further step into an aryl azoamino compound, the latter is reduced, the resulting diamine is transformed into the corresponding imidazole with phosgen or a reagent equivalent to phosgene, and, if desired, a protective group is introduced by reaction with substituted or unsubstituted, aliphatic or aromatic acid chlorides, aliphatic or aromatic carboxylic acid anhydrides, alkyl esters of halogenated formic acid, 1-alkoxyalkyl halides, enol ethers, aromatic or aliphatic sulfonic acid halides, diaryl phosphinic acid halides, phosphoric acid dialkyl ester halides, trialkyl silyl halides or trialkyl silyl acetamides.

3. Process according to claim 2, characterized in that as diazonium salt substituted benzene diazonium halides, borotetrafluorides or hydrogen sulfates are used.

4. Process according to one or both of claims 2 to 3, characterized in that as chiral amines the (R)- or (S)-1-phenylalkyl amines or the (R)- or (S)-2-aminophenyl acetic acid alkyl esters are used.

5. Process according to one or more of claims 2 to 4, characterized in that acidic catalysts are used for the transformation into the aryl azoamino compounds.

6. Process according to one or more of claims 2 to 5, characterized in that the reduction is carried out by catalytic hydrogenation with hydrogen or by reaction with zinc in the presence of hydrochloric acid or acetic acid.

7. Process according to claim 6, characterized in that the reduction is carried out by hydrogenation in the presence of platinum, palladium, rhodium, ruthenium or Raney-nickel, if desired, on carrier materials, such as carbon, alumina, pumice, aluminum oxide or aluminum silicate.

8. Process according to one or more of claims 2 to 7, characterized in that as phosgene equivalents compounds of the formula

YCOZ

are used, wherein Y und Z are the same and represent chlorine or imidazolyl, or Y is chlorine and Z is alkoxy, substituted or unsubstituied, or aryloxy, substituted or unsubstituted.

9. Process according to one or more of claims 2 to 8, characterized in that the reaction to imidazole is carried out in the presence of a base.

10. Process according to claim 9, characterized in that as base a tertiary amine is used.

## Revendications

1. Dérivés d'imidazole de formule

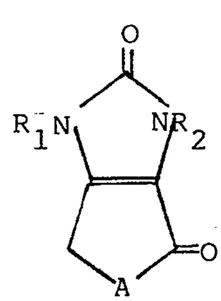

dans laquelle $R_1$ signifie un groupe (R)- ou (S)-1-phénylalkyle ($C_2$-$C_4$), un groupe (R)- ou (S)-1-alcoxy-($C_1$-$C_4$)carbonyl-1-phénylméthyle, un groupe (R)- ou (S)-1-phénoxycarbonyl-1-phénylméthyle ou un groupe (R)- ou (S)-1-benzyloxycarbonyl-1-phénylméthyle et $R_2$ représente l'hydrogène, un groupe

alkyl($C_1$-$C_4$) carbonyle éventuellement substitué par un halogène, un groupe benzoyle ou benzyle, chacun éventuellement substitué par des groupes alkyles inférieurs en $C_1$ à $C_6$ ou un halogène, un groupe alcoxy ($C_1$-$C_4$) carbonyle, un groupe phénoxycarbonyle, un groupe alcoxy ($C_1$-$C_4$)alkyle($C_1$-$C_4$), un groupe pyrannyle, un groupe benzènesulfonyle non substitué ou substitué par un groupe méthyle, un groupe alkyl($C_1$-$C_4$)sulfonyle, un groupe diphényl- ou dibenzylphosphinyle, un groupe dialcoxy($C_1$-$C_4$)phosphinyle ou un groupe trialkyl($C_1$-$C_4$)silyle et A représente un atome de soufre ou d'oxygène.

2. Procédé pour la préparation de dérivés d'imidazole selon la revendication 1, caractérisé en ce que l'on fait réagir un acide trétronique de formule

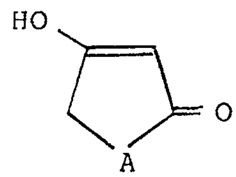

où A a la signification indiquée, avec un sel de diazonium, que l'on convertit dans une étape ultérieure l'acide arylazotétronique formé, respectivement l'arylhydrazone tautomère avec une amine chirale en un composé arylazoamino, réduit ce dernier, transforme la diamine formée avec du phosgène ou un réactif équivalent du phosgène en l'imidazole correspondant et introduit, le cas échéant, un groupe protecteur par réaction avec des chlorures d'acides aliphatiques ou aromatiques, substitués ou non substitués, des anhydrides d'acides carboxyliques aliphatiques ou aromatiques, des halogénoformiates d'alkyle, des halogénures de 1-alcoxyalkyle, des éthers énols, des halogénures de sulfonyle aromatiques ou aliphatiques, des halogénures de diarylphosphinyle, des halogénures d'esters dialkyliques d'acide phosphorique, des halogénures de trialkylsilyle ou des trialkylsilylacétamides.

3. Procédé selon la revendication 2, caractérisé en ce qu'on met en oeuvre en tant que sels de diazonium des halogénures, des borotétrafluorures ou des hydrogénosulfates de benzènediazonium substitués.

4. Procédé selon une ou deux des revendications 2-3, caractérisé en ce qu'on met en oeuvre en tant qu'amines chirales les (R)- ou (S)-1-phénylalkylamines ou les esters alkyliques d'acide (R)- ou (S)-2-aminophénylacétique.

5. Procédé selon une ou plusieurs des revendications 2-4, caractérisé en ce qu'on utilise des catalyseurs acides pour la conversion en les composés arylazoamino.

6. Procédé selon une ou plusieurs des revendications 2-5, caractérisé en ce que la réduction a lieu par hydrogénation catalytique avec de l'hydrogène ou par réaction avec du zinc en présence d'acide chlorhydrique ou d'acide acétique.

7. Procédé selon la revendication 6, caractérisé en ce que la réduction a lieu par hydrogénation en présence de platine, de palladium, de rhodium, de ruthénium ou de nickel de Raney, le cas échéant sur des supports tels que charbon, alumine, pierre ponce, oxyde d'aluminium ou silicate d'aluminium.

8. Procédé selon une ou plusieurs des revendications 2-7, caractérisé en ce qu'on met en oeuvre comme composés du phosgène des composés de formule

YCOZ

dans laquelle Y et Z sont identiques et signifient le chlore ou l'imidazoyle ou Y signifie le chlore et Z un alcoxy substitué ou non substitué ou un aryloxy substitué ou non substitué.

9. Procédé selon une ou plusieurs des revendications 2-8, caractérisé en ce qu'on effectue la conversion en imidazole en présence d'une base.

10. Procédé selon la revendication 9, caractérisé en ce qu'on utilise une amine tertiaire en tant que base.